# EUROPEAN PATENT SPECIFICATION

(11) **EP 0 519 140 B1**
(45) Date of publication and mention of the grant of the patent: **29.07.1998**
(21) Application number: 91401662.1
(22) Date of filing: 19.06.1991
(51) Int. Cl.: C07D 237/28, A01N 43/58

(54) **Method for the preparation of substituted 1,4-dihydro-4-oxo-cinnoline-3-carboxylic acids, esters and salts thereof, and intermediates used in this method**
Verfahren zur Herstellung von substituierten 1,4-Dihydro-4-oxo-cinnolin-3-carbonsäuren, ihre Ester und Salze, und in diesem Verfahren benutzten Zwischenprodukte
Procédé pour la préparation d'acides 1,4-dihydro-4-oxo-cinnoline-3-carboxyliques substitués, leurs esters et sels, et produits intermédiaires utilisés dans ce procédé

(43) Date of publication of application: 23.12.1992
(73) Proprietor: HYBRINOVA, F-91953 Les Ulis Cedex (FR)
(72) Inventor: Labovitz, Jeffrey, Palo Alto, California 94301 (US); Guilford, William J., San Leandro, California 94578 (US); Fang, Lawrence, Foster City, California 94404 (US); Liang, Yi, San Jose, California 95129 (US)
(74) Representative: Phélip, Bruno

(56) References cited:
- EP-A- 138 661
- EP-A- 287 853
- EP-A- 320 793
- EP-A- 363 236
- CHEMICAL ABSTRACTS, vol. 65, no. 9, 24 October 1966, Columbus, Ohio, US; abstract no. 13596H, E. PROFFT ET AL.: 'ACID DERIVATIVES OF TETRACHLOROPHTHALIC ACID.' & ARCHIV DER PHARMAZIE vol. 299, no. 7, 1966, WEINHEIM DE, pages 577 - 588

## Description

### BACKGROUND OF THE INVENTION

### Field Of The Invention

The present invention relates to a method for the preparation of certain substituted cinnoline derivatives, to new chemical intermediates used to prepare such cinnoline derivatives and to a method for the preparation of said intermediates. The substituted cinnoline derivatives have utility in regulating the fertility of certain plants.

### Description Of The Background

Cross-breeding of plants has been commercially used for decades to alter the genetic make-up of plants. For successful cross-breeding, it is necessary to prevent the occurrence of self-pollination. In plants such as corn, self-pollination is prevented through mechanical means, as by removing the tassel which is the male portion of the plant and thus its pollen source. However, in other plants, such as wheat, the physiology of the plant prevents mechanical means from being successfully used to prevent self-pollination. In such plants, the male part is inaccessibly located in close proximity to the female part, making the use of mechanical intervention impractical.

Prevention of self-pollination in plants such as wheat requires a chemical means to suppress the formation of active pollen. Certain chemical pollen suppressants have already been suggested in the art, as discussed, for example, in U.S. Patent Nos. 4,561,881, 4,604,134, 4,729,782, and 4,756,740 and in EP 0.138.661 and the references discussed therein. Such pollen suppressants chemically inhibit the formation of pollen or induce the plant to produce non-functioning pollen. More recently, it has been discovered that certain 5-oxy or 5-amino substituted 1,4-dihydro-4-oxo-cinnoline-3-carboxylic acids and esters and salts thereof have superior pollen suppression activity, as disclosed in U.S. Patent Application Serial No. 243,895, filed September 13, 1988 and in EP 0.363.236. The chemical pollen suppressants of these applications are species of compounds having the formula: 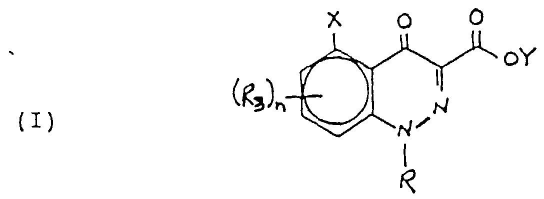 wherein
X represents a group of the formula OR₁ or NR₁R₂ wherein R₁ represents a C₁-C₄ alkyl (optionally substituted with a C₁-C₄ alkoxy group, 1 to 3 halogen atoms, or a carboxy or C₁-C₄ alkoxycarbonyl group), a C₂-C₄ alkenyl (optionally substituted with 1 to 3 halogen atoms), or a C₂-C₄ alkynyl group and R₂ represents H or an alkyl group;
Y is hydrogen, C₁-C₂₂ linear alkyl or alkenyl containing up to four carbon-carbon double bonds, C₃-C₆ branched alkyl or alkenyl, C₁-C₄ alkoxyalkyl, cyclohexylmethyl, halogenated C₁-C₄ alkyl, phenyl, benzyl, -(CH₂CH₂O)ₘCH₂CH₃ in which m is an integer from 1 to 5, or -CH(CH₂OR₄)CH₂OR₅ or -CH₂CHOR₄CH₂OR₅ in which either R₄ or R₅ but not both represent a C₁-C₂₂ linear alkyl- or alkenylcarbonyl group containing up to four carbon-carbon double bonds and the other of R₄ or R₅ is H;
R represents C₁-C₄ alkyl, phenyl, naphthyl, or phenyl or naphthyl substituted with one to three substituents selected from the group consisting of halogen, CONH₂, C₁-C₄ alkyl or haloalkyl, C₁-C₄ alkoxy or haloalkoxy, and cyano; and
each R₃ is C₁-C₄ alkyl or haloalkyl, C₁-C₄ alkoxy or haloalkoxy, C₁-C₄ alkanoyl, cyano, nitro, hydroxy, or halo substituent which can be the same or different and n is an integer from 0 to 3;
and salts thereof.

Unfortunately, the chemical pollen suppressants disclosed in the aforementioned applications are somewhat difficult to prepare and require the use of expensive starting materials. In particular, the compounds disclosed in these applications have typically been synthesized by reacting difluorobenzoyl chloride and derivatives thereof with monoethyl malonate. This process requires a distillation step. Furthermore, the yield of this process is only 67%. The difluorobenzoyl chloride and its derivatives have the formula: 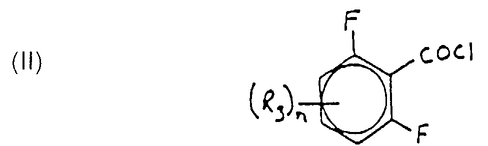 wherein R₃ and n have the previously defined meanings.

Fluorinated compounds such as those of the foregoing formula II are very expensive raw materials as are the solvents (such as DMAP) in which these compounds are normally reacted. Thus, while synthesis of compounds of Formula I was possible in the laboratory, commercial production in large scale was economically unfeasible.

It is apparent that a need exists for a procedure for synthesizing compounds of Formula I which does not require the use of expensive or difficult-to-obtain starting materials and solvents. Heretofore, that need has not been satisfied by any of the known synthesis routes for manufacturing such compounds.

### SUMMARY OF THE INVENTION

Surprisingly, there has now been discovered an efficacious method for the preparation of the substituted 1,4-dihydro-4-oxo-cinnoline-3-carboxylic acids and esters and salts thereof of Formula I which avoids the need for expensive or unavailable raw materials and the need for extreme reaction conditions. The new method uses less-expensive, readily available starting materials to produce a new chemical intermediate which is unknown in the art and which has never been used to synthesize these compounds.

In accordance with the present invention certain new intermediates are first synthesized from readily available, relatively inexpensive starting materials by a process which comprises reacting a chlorobenzoyl compound (preferably a benzoyl chloride) or a derivative thereof with a ketocarboxylate, wherein the chlorobenzoyl compound and derivatives thereof have the following formula: 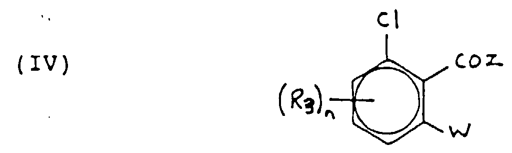 wherein
R₃ and n have the previously defined meanings;
W is either F or Cl; and
Z is H or Cl.

The ketocarboxylate which is reacted with the dichlorobenzoyl compound of formula IV has the following formula: 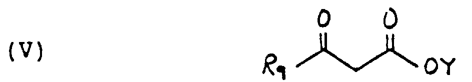 wherein Y has the previously defined meanings, and R₉ is a hydrocarbon group containing 10 or fewer carbons, especially one in which an alkyl group is present adjacent to the β-keto group, with R₉ preferably being entirely a short-chain hydrocarbon, such as an alkyl or alkenyl group having from 1 to 4 carbon atoms, more preferably a methyl group.

Reaction of the dichlorobenzoyl compound of formula IV with the ketocarboxylate of formula V results in the formation of a 2,6-dichlorobenzoylketocarboxylate or trione having the following formula: 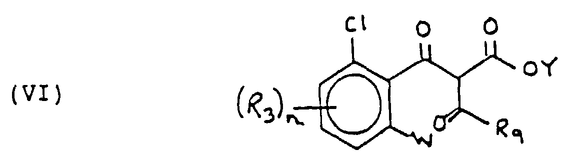 wherein R₃, R₉, W, Y , and n have the previously defined meanings.

Through further chemical conversion, using procedures known in the art, the compounds of Formula VI may be readily transformed into the desired compounds of Formula I and related compounds. The compounds of Formula VI are new chemical entities that provide a new synthetic route to substituted cinnolines as described herein.

One trione, or 2,6-dichlorobenzoylketocarboxylate, has been described by Profft et al. (Chemical Abstracts, vol.65, n°9, 1966, abstract n°13 596 H). This document does not disclose the preparation of substituted cinnoline derivatives.

### DESCRIPTION OF SPECIFIC EMBODIMENTS

The present invention provides a novel process for the preparation of substituted 1,4-dihydro-4-oxo-cinnoline-3-carboxylic acids, esters, and salts thereof in which an oxy or amino substituent is present at position 5 of the derivatized cinnoline ring. A key step in the process is the reaction of a chlorobenzoyl compound with a keto carboxylate. The resulting triones not only represents a new synthetic route to cinnolines, they are novel compounds in and of themselves. Thus, the present invention provides a process for the preparation of certain chemical pollen suppressants and other compounds of the formula: 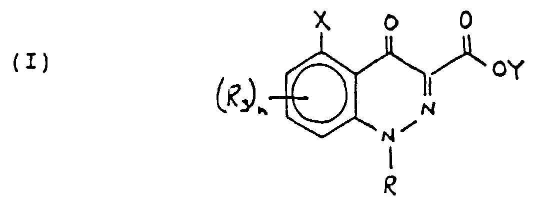 wherein
X represents a group of the formula OR₁ or NR₁R₂ wherein R₁ represents a C₁-C₄ alkyl (optionally substituted with a C₁-C₄ alkoxy group, 1 to 3 halogen atoms, or a carboxy or C₁-C₄ alkoxycarbonyl group), a C₂-C₄ alkenyl (optionally substituted with 1 to 3 halogen atoms), or a C₂-C₄ alkynyl group and R₂ represents H or an alkyl group;
Y is hydrogen, C₁-C₂₂ linear alkyl or alkenyl containing up to four carbon-carbon double bonds, C₃-C₆ branched alkyl or alkenyl, C₁-C₄ alkoxyalkyl, cyclohexylmethyl, halogenated C₁-C₄ alkyl, phenyl, benzyl, -(CH₂CH₂O)ₘCH₂CH₃ in which m is an integer from 1 to 5, or -CH(CH₂OR₄)CH₂OR₅ or -CH₂CHOR₄CH₂OR₅ in which either R₄ or R₅ but not both represent a C₁-C₂₂ linear alkyl- or alkenylcarbonyl group containing up to four carbon-carbon double bonds and the other of R₄ or R₅ is H;
R represents C₁-C₄ alkyl, phenyl, naphthyl, or phenyl or naphthyl substituted with one to three substituents selected from the group consisting of halogen, CONH₂, C₁-C₄ alkyl or haloalkyl, C₁-C₄ alkoxy or haloalkoxy, and cyano;
each R₃ is C₁-C₄ alkyl or haloalkyl, C₁-C₄ alkoxy or haloalkoxy, C₁-C₄ alkanoyl, cyano, nitro, hydroxy or halo, and most preferably hydrogen, and n is an integer from 0 to 3;
and salts thereof.

The present invention relates also to compounds of formula (VI) having the above described R₃, W, Y and R₉ substituents, with the proviso that such a compound cannot be p-C₆Cl₄(COCHA_{c}CO₂Et)₂.

### General Synthesis Route

The present invention provides a method for the preparation of the substituted cinnoline derivatives of Formula I, through the following reaction sequence. In this reaction scheme, substituents not involved in the actual reaction being shown have the meanings previously indicated.

### Exemplary Step A:

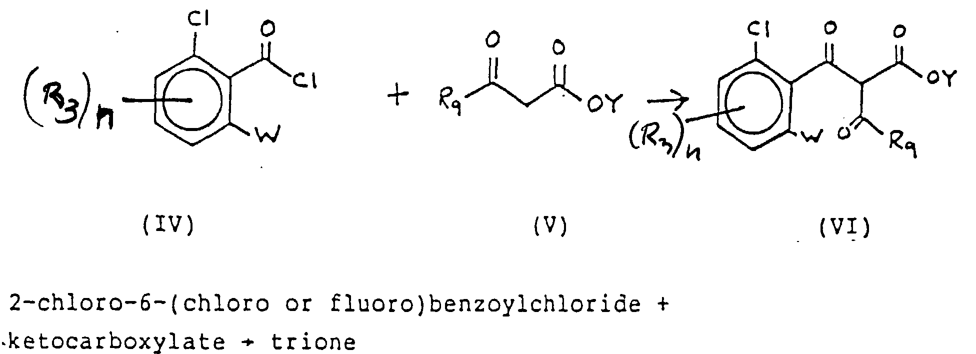

### Exemplary Step B:

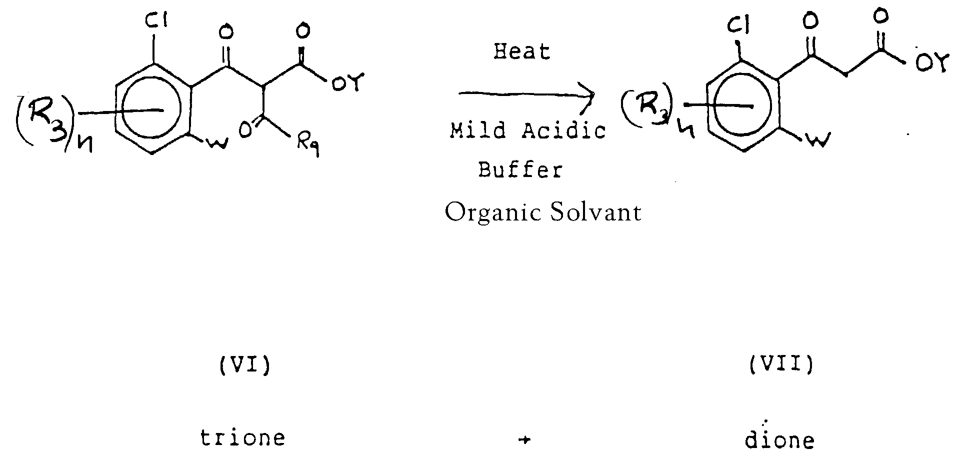

### Exemplary Step C:

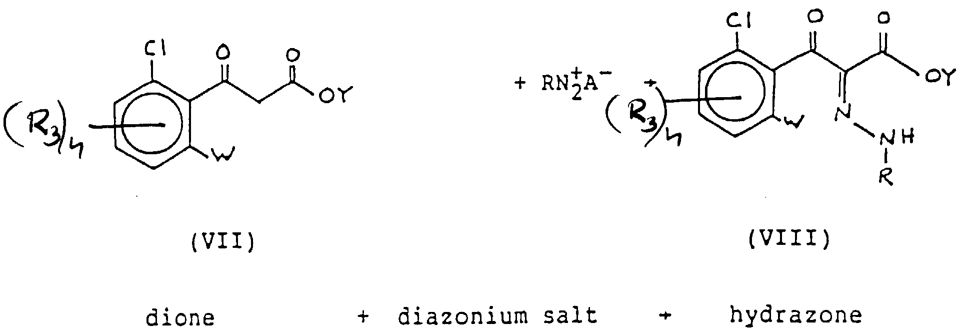

A is a counter anion

### Exemplary Step D:

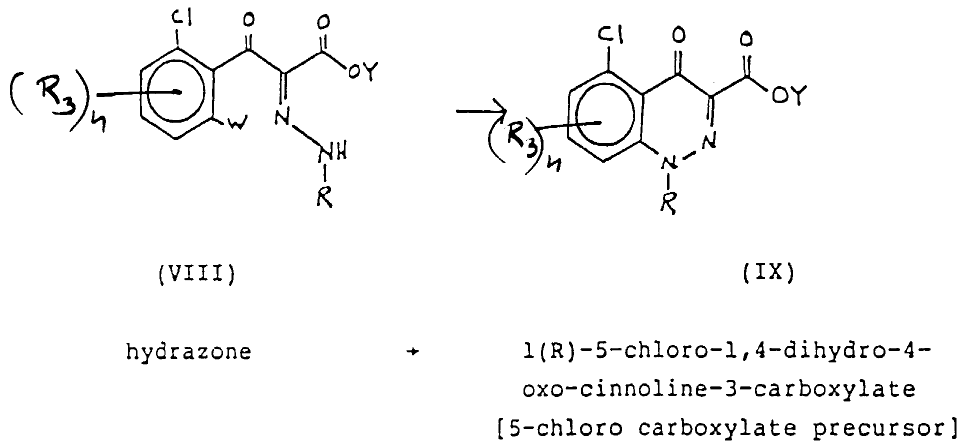

### Exemplary Step E (optional):

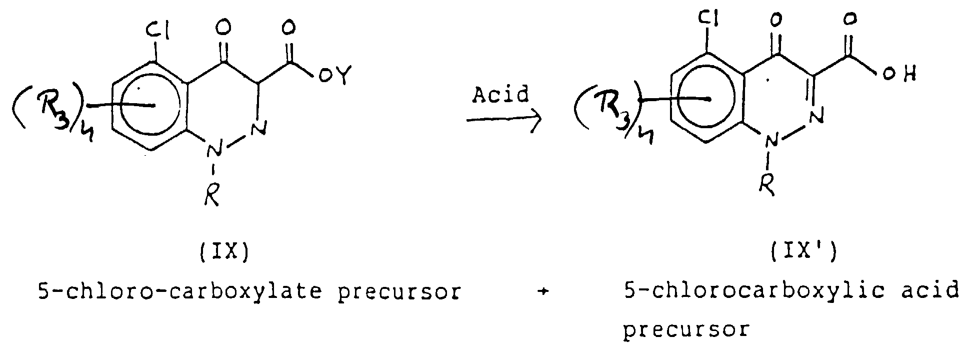

### Exemplary Step F:

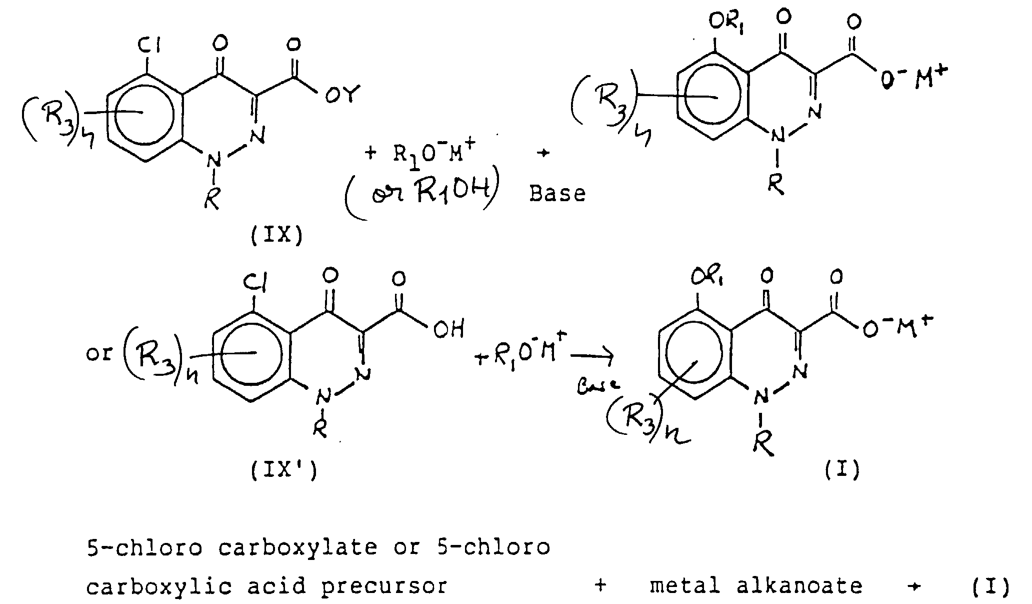

M is a metal cation

### Exemplary Step G:

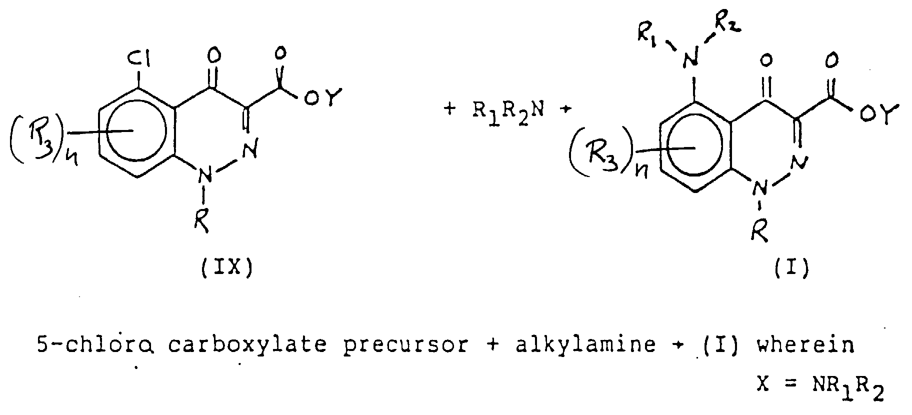

Steps A and B above are new synthesis procedures which are not found in the prior art in the synthesis of compounds of Formula I, whereas Steps C through G are generally well-known procedures which have been used previously in the art in the synthesis of compounds of Formula I.

Step A not only is a new reaction, but the 2-(2',6'-dihalobenzoyl)-3-ketocarboxylate, or trione, product of that reaction is a new chemical entity. Such a trione can be the 2-(2',6'-dichlorobenzoyl)-3-keto-3-(C₁-C₄ alkyl) propanoic acid ester, or 2-(2',6'-dichlorobenzoyl)-3-ketobutanoic acid ester.

Likewise, the reaction of Step B also is known in the production of substituted cinnolines, and in combination with Step A affords a method for obtaining a 2,6-dihalobenzoyl carboxylate which previously could only be obtained through use of more expensive starting materials and extreme laboratory conditions.

Steps B and C can be combined by reacting the trione directly with the diazonium salt (Japp-Klingman reaction).

### Specific Reaction Conditions

Although, in general, the reaction conditions used in the practice of the method of the present invention are not critical, certain specific conditions yield preferred results in terms of the yield or purity of the desired products, their rate of formation, or other considerations. On that basis, the preferred reaction conditions for each of the steps are set forth below.

### Step A: Formation of 2,6-dichlorobenzoyl ketocarboxylate-trione

Generally from about 1 to about 3 moles of the ketocarboxylate is employed per mole of 2,6-dichlorobenzoyl chloride or derivatives thereof. Preferably an aprotic solvent is used, most preferably one selected from the group consisting of toluene/methylcyanide (at a usual volume ratio of about 2:1), methylcyanide, tetrahydrofuran (THF); or THF/methylcyanide (1:2). The reaction temperature is from about 60 to about 90°C, and the reaction takes place in the presence of a catalytic amount of a base such as pyridine and magnesium chloride and generally proceeds for about 3 to about 8 hours.

### Step B: Formation of 2,6-dichlorobenzoyl carboxylate -- Dione

The 2,6-dichlorobenzoyl ketocarboxylate is converted to the corresponding 2,6-dichlorobenzoyl carboxylate by heating the starting compound in an organic solvent at a temperature from about 50 to about 100°C for about 6 to about 36 hours. Preferably, the organic solvent is an alcohol such as methanol. The conversion reaction takes place most readily if the reaction mixture is buffered, preferably at a pH from about 4 to about 7. The preferred buffer is potassium hydroxide/acetic acid, although other buffers such as potassium phosphate can also be used. Preferably the ratio of the molarity of the buffer to the molarity of the starting carboxylate is from about 10:1 to about 2:1. After completion of the reaction, an appropriate organic solvent, such as methylene chloride, can be used as a vehicle for the entire reaction mixture, which then can be suitably washed with water to remove the buffer, followed by drying of the organic layer and vacuum removal of the solvent to recover the desired product.

### Step C: Formation of Hydrazone

A diazonium salt is first prepared by means known in the art, typically by reacting an amine of the formula RNH₂ with sodium nitrite by first adding the amine to concentrated acid, such as hydrochloric, to form an amine salt which subsequently is combined with an aqueous solution of sodium nitrate. The resultant diazonium salt is then added to the 2,6-dichlorobenzoyl carboxylate in a suitable organic solvent, such as methanol, containing sodium acetate. The desired hydrazone precipitates from solution and can then be collected by filtration, followed by washing with water and vacuum drying.

### Step D: Formation of 1(R)-5-chloro-1,4-dihydro-4-oxo-cinnoline-3-carboxylate -- 5-chloro carboxylate precursor

About 1 to about 2 equivalents of a base such as potassium carbonate per mole of hydrazone is combined in an anhydrous solvent, such as dimethylformamide, along with a catalytic amount of a crown ether, such as 18-crown-6. The resulting mixture is then warmed to a temperature of about 110 to about 150°C for about 1/2 to about 4 hours, under a nitrogen atmosphere. After cooling, the reaction mixture is poured into water with stirring, and the desired 5-chloro carboxylate precursor removed.

### Step E: Formation of 1(R)-5-chloro-1,4-dihydro-4-oxo-cinnoline-3-carboxylic acid -- 5-chloro carboxylic acid precursor

The 5-chloro carboxylate precursor is placed in an appropriate solvent such as p-dioxane which is then acidified with a strong acid such as hydrochloric acid. The mixture is then refluxed for about 4 hours to about 12 hours to complete the saponification reaction, after which the mixture is cooled and poured into water and the 5-chloro carboxylic acid precursor precipitate filtered and dried under vacuum.

### Step F: Formation of 1(R)-5-(oxy substituted)-1,4-dihydro-4-oxo-cinnoline-3-carboxylic acid

Either the 5-chloro carboxylate precursor or the 5-chloro carboxylic acid precursor is placed in a suitable organic solvent such as tetrahydrofuran (THF), dimethyl formamide (DMF), p-dioxane, or methanol, to which a base, such as potassium hydroxide, is added, along with an alcohol having the formula R₁OH, wherein R₁ has the previously defined meaning to form the corresponding potassium alkoxide. The resultant mixture is refluxed for about 8 to about 16 hours, then cooled and acidified and the product recovered as the salt or, if acidified before recovery, as the free acid, by filtration and drying over vacuum.

### Step G: Formation of 1(R)-5-(amino substituted)-1,4-dihydro-4-oxo-cinnoline-3-carboxylic acid

The desired alkylamine or dialkylamine R₁R₂N wherein R₁ and R₂ have the previously defined meanings (10 equivalents) is added to a solution or suspension of the starting 5-chloro-4-oxo-cinnoline-3-carboxylic acid precursor in aqueous dioxane or another suitable solvent. The mixture is refluxed overnight. The reaction is followed by HPLC, and, upon completion, the mixture is diluted with two volumes of cold water or another miscible liquid in which the product is less soluble. After the pH is adjusted to the isoelectric point, the product precipitates and is collected by filtration, washing with water, and drying.

### Description of Preferred Constituents

In one preferred embodiment of the invention, -CO₂Y is a carboxy group or a salt thereof. When -CO₂Y is a salt of a carboxy group, the cation can be an alkali metal ion, alkaline earth metal ion, or transition metal ion. The cation can also be an ammonium or substituted ammonium ion. Representative alkali metal ions, which are preferred, include lithium, sodium, and potassium ions; representative alkaline earth metal ions include magnesium, calcium, and barium ions; representative transition metal ions include zinc, manganese, iron, titanium, and molybdenum ions; and representative ammonium ions, which are also preferred, include the ammonium ion itself and alkyl-substituted ammonium ions (especially alkanol-substituted ammonium ions).

Preferred substituents at other positions are those in which R represents a phenyl group with zero to three substituents selected from the group consisting of halogen, -CONH₂, C₁-C₄ alkyl or haloalkyl (especially trihalomethyl), C₁-C₄ alkoxy or haloalkoxy, and cyano ("haloalkyl" and similar terms referring to halogenated molecules include both mono- and polyhalogenated compounds); more preferably the phenyl is substituted with one of said substituents; even more preferably with one halogen atom; and most preferably with chlorine in the para position; Y is H, Na, K, C₁-C₂₂ linear alkyl or alkenyl containing up to four carbon-carbon double bonds, C₁-C₄ alkoxyalkyl, cyclohexylmethyl, halogenated C₁-C₄ alkyl, phenyl, benzyl, -(CH₂CH₂O)ₘCH₂CH₃ in which m is an integer from 1 to 5, or -CH(CH₂OR₄)CH₂OR₅ or -CH₂CHOR₄CH₂OR₅ in which either R₄ or R₅ but not both represents a C₁-C₂₂ linear alkyl or alkenyl group containing up to four carbon-carbon double bonds (the other of R₄ or R₅ representing hydrogen); and X represents a group of the formula OR₁ or NR₁R₂ wherein the R₁ represents a C₁-C₄ alkyl (optionally substituted with a C₁-C₄ alkoxy group, 1 to 3 halogen atoms, or a carboxy or C₁-C₄ alkylcarbonyl group), alkenyl, or alkynyl group and R₂ represents H or CH₃, with the proviso that when R₁ is CH₃, R₂ is CH₃.

Among the preferred esters of the carboxylic acid group at position 3 of the cinnoline ring are esters formed from fatty alcohols or from fatty acid monogylcerides. Examples of fatty alcohols include arachidyl, cetyl, decanol, lauryl, linolenyl, linoleyl, and oleyl alcohols. Examples of fatty acids include the fatty acids corresponding to these fatty alcohols. The fatty acids are typically reacted first with glycerin to form monoglycerides (the reaction occurring at either the 1 or the 2 position), with the resulting monoglyceride being reacted at either of the free hydroxyl groups to form the final ester. Other preferred esters include those prepared from linear and branched C₁-C₆ alkanols.

Another grouping of preferred substituents includes those in which the substituents on a phenyl group in the R position are selected from the group consisting of 3',4'-dichloro, 3',4'-difluoro, 4'-trifluoromethoxy, 4'-chloro, 4'-methyl, 4'-methoxy, 4'-cyano, 4'-trifluoromethyl, 4'-iodo, 4'-fluoro, 4'-bromo, 3'-fluoro, 3'-chloro, 3'-trifluoromethyl, 2'-fluoro, 2'-chloro, and 2'-trifluoromethyl. At the Y location, tetrabutyl ammonium and tetramethyl ammonium salts are especially preferred along with ammonium salts containing alkanol substituents in place of alkyl substituents. Preferred -CO₂Y groups are acids and acid salts, although esters as described above are nearly as preferred. Among acid salts, quaternary ammonium salts are preferred, as they enhance stability. The OR₁ substituent is preferably -OMe, -OEt, -OnPr, -OiPr, -OCH₂CH=CH₂, -OiBu, -OCH₂CH₂OCH₃, -OCH₂CH₂OCH₂CH₃, -NMe₂ or -OCH(CH₃)CO₂H.

Certain combinations of substituents are especially preferred. One preferred group occurs when R is phenyl or phenyl mono-substituted with a halogen, trihalomethyl, or cyano group at position 4' or a halogen at position 2'; Y is -H, -Na, or K; and OR₁ represents -OMe, -OEt, -OnPr, -OiPr, -OCH₂CH=CH₂, -OiBu, -NMe₂, -OCH₂CH₂OCH₃, or- OCH₂CH₂OCH₂CH₃.

Other preferred groups occur when:
R is phenyl or phenyl substituted with 4'-fluoro or 2'-fluoro, X is OCH₃, and Y is H or cation of a salt;
R is phenyl or phenyl substituted with 3',4'-difluoro, X is OCH₃, and Y is H or cation of a salt;
R is phenyl, X is OCH₂CH₂CH₃ or OCH(CH₃)₂ and Y is H or cation of a salt;
R is phenyl or phenyl substituted with 4'-chloro, X is OCH₂CH₂OCH₃, and Y is H or cation of a salt;
R is phenyl or phenyl substituted with 4'-chloro, X is OCH(CH₃)CO₂H, and Y is H or cation of a salt.

Also included within the scope of the invention is the formation of agronomically acceptable acid addition salts of compounds having the general formula I. Typical acid addition salts are formed with strong acids such as hydrochloric, hydrobromic, and sulfuric acids. Salts of acidic or basic functional groups, such as the -CO₂Y or -X groups, may also be formed in this invention. Throughout this application, agronomically acceptable salt means that the salt is not substantially more toxic to the plant or to the consumer of the plant than the parent compound from which the salt is formed.

Typical compounds produced by the process of the invention include the following:
1-phenyl-1,4-dihydro-4-oxo-5-methoxycinnoline-3-carboxylic acid
1-(4'-chlorophenyl)-1,4-dihydro-4-oxo-5-(1"-carboxy-ethoxy)cinnoline-3-carboxylic acid
1-(4'-fluorophenyl)-1,4-dihydro-4-oxo-5-ethoxycinnoline-3-carboxylic acid
1-phenyl-1,4-dihydro-4-oxo-5-(2"-methoxyethoxy)-cinnoline-3-carboxylic acid
1-(4'-chlorophenyl)-1,4-dihydro-4-oxo-5-n-propyloxycinnoline-3-carboxylic acid
1-(4'-trifluoromethyl)-1,4-dihydro-4-oxo-5-dimethylaminocinnoline-3-carboxylic acid
1-(3',4'-dichlorophenyl)-1,4-dihydro-4-oxo-5-methoxycinnoline-3-carboxylic acid
1-(4'-cyanophenyl)-1,4-dihydro-4-oxo-5-(prop-2"-enoxy)-cinnoline-3-carboxylic acid
1-(4'-fluorophenyl)-1,4-dihydro-4-oxo-5-i-butoxycinnoline-3-carboxylic acid
1-(3'-chlorophenyl)-1,4-dihydro-4-oxo-5-dimethylaminocinnoline-3-carboxylic acid
1-(4'-trifluoromethylphenyl)-1,4-dihydro-4-oxo-5-methoxycinnoline-3-carboxylic acid
as well as the ammonium, sodium, potassium, and lithium carboxylate salts of each of the above compounds and the acid addition salts of each of the above listed compounds. By carboxylate salt is meant a salt of a carboxylic acid group at C-3. By acid addition salt is meant a salt formed by the protonation of a ring or side chain nitrogen.

Aromatic substituents, R₃, may also be present. Groups that would interfere with the ring-forming reaction or other reactions may be present in protected form (e.g., an acylamino group that may later be converted into an amine), or they may be added later (e.g., by halogenation of the phenyl rings), or they may be prepared by conversion of a suitable group present during synthesis (e.g., the above-mentioned amino group may be diazotized and converted into many different functional groups).

The above-indicated 3-carboxycinnolines can then be converted into other compounds by known methods. For example, the carboxylic acid group can be converted into a carboxylate salt or a protected amino group can be deprotected, diazotized, and converted into a different functional group by simple synthetic manipulations within the ordinary skill of a synthetic organic chemist.

Having now generally described this invention, the same will be better understood by reference to certain specific examples which are included herein for purposes of illustration only and are not intended to be limiting of the invention or any embodiment thereof unless so specified.

### Examples I(a)-I(i)

### Preparation of Methyl 2,6-Dichlorobenzoylacetoacetate

**I(a)** To a stirred slurry of 190 g (2 moles) of magnesium chloride in 1 liter of acetonitrile is added 232 g (2 moles) of methyl acetoacetate, the reaction is cooled to 0°C, and there is then added 324 ml (4 moles) of pyridine dropwise such that the temperature does not exceed 5°C.

After the mixture is stirred at room temperature for 30 minutes, 419 g (2 moles) of 2,6-dichlorobenzoyl chloride in 1 liter of toluene is added and the reaction heated to reflux for 4 hours (85-90°C).

The reaction is then cooled to 0°C, and there is slowly added 133 ml (2.4 moles) of concentrated sulfuric acid. The mixture is extracted with water/methylene chloride to separate the aqueous layers (pH = 1), washed with water once (pH = 4), dried and the solvent evaporated in vacuo to give 535 g (92.5%) of the crude trione.

**I(b)-I(i)** Following the general procedure of Example I(a), Examples I(b)-I(i) are prepared using different solvents and using principal reaction conditions of 65-85°C, for 3 to 8 hours. The amount of acetoacetate employed is set forth in Table 1, along with the purity and yield of the resulting product where available. In some cases (indicated by a "-" at the appropriate location in the following Tables), data was not obtained on purity and yield.

**TABLE 1**

| **Summary Of Reaction Conditions And Results For Preparation Of Methyl Dichlorobenzoylacetoacetates** | | | | |
|---|---|---|---|---|
| RXN | Solvent | [Acetoacetate]¹ | (%) Purity | (%) Yield |
| I(b) | p-Dioxane | 0.25 | - | - |
| I(c) | Monoglyme | 0.25 | - | - |
| I(d) | Diglyme | 0.25 | - | - |
| I(e) | C₆H₅CH₃/CH₃CN (2:1) | 0.50 | 80 | 87 |
| I(f) | CH₃CN | 0.25 | 78 | 87 |
| I(g) | THF/CH₃CN (1:2) | 0.50 | 68 | 33 |
| I(h) | THF | 0.50 | 91 | 93 |
| I(i) | THF | 1.67 | 85 | 88 |

| | | | | |
|---|---|---|---|---|
| ¹ Moles acetoacetate per liter of solvent | | | | |

### Examples II(a)-II(h)

### Preparation of Methyl 2,6-Dichlorobenzoylacetate

**II(a)** There is prepared 1.85 M KOAc/MeOH/AcOH buffer by dissolving 544 g (5.55 moles) of potassium acetate in 2.21 of methanol and adding 570 ml of acetic acid to reach pH = 6.5 and a final volume of 3.0 l.

The above buffer is added to the crude trione of Example I (1.85 moles) and heated to a reflux for 16-20 hours (65-70°C). The reaction can be monitored by HPLC to assure completion.

After being cooled to room temperature, the mixture is taken up in methylene chloride and washed with water to remove buffer (pH = 5-6). The organic layer is dried and solvent is removed in vacuo to afford 365 g (80%) of the beta-keto ester with a purity of 90%.

**II(b)-II(h)** Following the general procedure of Example II(a), Examples II(b)-II(h) are performed using different reagents and pH conditions, using principal reaction conditions of 50-65°C, for 16 to 24 hours. The nature and amount of reagent employed and pH are set forth in Table 2, along with the purity and yield of the resulting products where available.

**TABLE 2**

| **Summary Of Reaction Conditions And Results For Preparation Of Methyl Dichlorobenzoylacetates** | | | | | |
|---|---|---|---|---|---|
| RXN | Reagents | [Reagent] | pH | (%) Purity | (%) Yield |
| II(b) | Zn(OAc)₂.2H₂O | 1.50 | 5.8 | - | - |
| II(c) | Zn(OAc)₂.2H₂O | 0.01 | 7.3 | - | - |
| II(d) | AcOH | 10.00 | 0.8 | - | - |
| II(e) | ZnCl₂ | 1.25 | 4.5 | 82 | 46 |
| II(f) | KOAc/AcOH | 1.58 | 7.5 | 74 | 40 |
| II(g) | KOAc/AcOH | 1.41 | 6.4 | 86 | 46 |
| II(h) | KOAc/AcOH | 1.31 | 5.3 | 83 | 54 |

### Examples III(a)-III(f)

### Preparation of 1-(4'-chlorophenyl)-5-methoxyethoxy-1,4-dihydro-4-oxo-cinnoline-3-carboxylic acid

### III(a)

### 1. Preparation of methyl 3-(2,6-dichlorophenyl)-2,3-dioxopropionate-2-(4'-chlorophenyl) hydrazone

To 13.4 g (0.105 mole) of 4-chloroaniline in 80 ml of methanol was added 33.4 ml (4.0 eq.) of concentrated HCl with ice water cooling. There was then added at 0-5°C 7.2 g (0.105 mole) of NaNO₂ in 20 ml of H₂O with vigorous stirring. The resulting solution of diazonium salt is then added with continued stirring at 5-10°C to a solution of methyl 2,6-dichlorobenzoylacetate (24.7 g) in 150 ml of MeOH containing 29.7 g (3 eq.) of potassium acetate. The resulting precipitate is collected by filtration, washed with H₂O, and dried over vacuum at 50°C (28.6 g, 74%).

### 2. Preparation of methyl 1-(4'-chlorophenyl)-5-chloro-1,4-dihydro-4-oxo-cinnoline-3-carboxylate

To 12 g (31 mmole) of the above hydrazone (from step 1) in 150 ml of dry dimethyl formamide are added 4.3 g (1.0 eq.) of K₂CO₃ and a catalytic amount of 18-crown-6 at room temperature. The mixture is warmed to 130°C for 1 hour, under a nitrogen atmosphere. After cooling, the mixture is poured into water with stirring. 10 g (92.4%) of crude product is made.

### 3. Preparation of 1-(4'-chlorophenyl)-5-chloro-1,4-dihydro-4-oxo-cinnoline-3-carboxylic acid

To 10 g (28.6 mmole) of the above methyl ester (from step 2) in 150 ml of p-dioxane there is added 10 ml (4 eq.) of concentrated HCl with stirring under a nitrogen atmosphere. The mixture is heated up and refluxed for 5 hours. After cooling, the mixture is poured into water with stirring. Free acid is collected by filtration and dried over vacuum at 50°C (9.5 g, 99%).

### 4. Preparation of 1-(4'-chlorophenyl)-5-methoxyethoxy-1,4-dihydro-cinnoline-4-oxo-3-carboxylic acid

To 0.5 g (1.5 mmole) of the above free acid (from step 3) in 20 ml of THF there is added a mixture of 0.8 g (8 eq) of KOH in 5 ml of 2-methoxy-ethanol at room temperature. The entire mixture is refluxed for 5 hours, cooled, and acidified with 1 N HCl. The product is filtrated and dried over vacuum at 50°C (0.52 g, 93%).

**III(b)-III(f)** Following the general procedure of Example III(a), Examples III(b)-III(f) are performed using different solvents, reaction temperatures, and reaction times, all as set forth in Table 3. Also set forth in that table are the purity and yields obtained in each instance where available.

**TABLE 3**

| **Summary of Reaction Conditions And Results For Making 1-(4'-chlorophenyl)-5-methoxyethoxy 1,4-dihydro-4-oxo-cinnoline-3-carboxylic Acid** | | | | | |
|---|---|---|---|---|---|
| Eq. of KOH | (°C) rxn Temp. | Solvent | (hrs.) rxn Time | (%) Purity | (%) Yield |
| III(b) | 75 | p-dioxane | 2 | 95 | 89 |
| III(c) | 85 | p-dioxane | 3 | 89 | 90 |
| III(d) | 100 | p-dioxane | 15 | 96 | 92 |
| III(e) | 100 | THF | 4 | 96 | 93 |
| III(f) | room | DMF | 15 | - | - |

### Examples IV(a)-IV(f)

### Preparation of 1-(4'-fluorophenyl)-5-methoxy-1,4-dihydro-4-oxo-cinnoline-3-carboxylic acid

### IV(a)

### 1. Preparation of methyl 3-(2,6-dichlorophenyl)-2,3-dioxo-propionate-2-(4'-fluorophenyl) hydrazone

To 10.55 g (95 mmole) of 4-fluoroaniline in 80 ml of methanol there is added 33.3 ml (4 eq.) of concentrated HCl with ice water cooling. There is then added at 0-5°C, 6.5 g (95 mmoles) of NaNO₂ in 20 ml of H₂O, while stirring is vigorously maintained. The resulting solution of diazonium salt is then added with stirring at 5-10°C to a solution of methyl 2,6-dichlorobenzoylacetate(24.7 g, 90 mmole) in 150 ml of MeOH containing 29.2 g (3.0 eq) of potassium acetate. The resulting precipitate is collected by filtration, washed with H₂O, and dried over vacuum at 50°C (21.2 g, 57%).

### 2. Preparation of methyl 1-(4'-fluorophenyl)-5-chloro-1,4-dihydro-4-oxo-cinnoline-3-carboxylate

To the above 21.2 g (57.4 mmole) of hydrazone in 180 ml of dry DMF there is added 7.94 g (1 eq) of K₂CO₃ and a catalytic amount of 18-crown-6 at room temperature. The mixture was warmed up to 130°C for 1 hour under N₂ atmosphere. After cooling, the mixture is poured into water with stirring. The product is collected by filtration, washed with H₂O, and dried over vacuum at 50°C (18.5 g, 97%).

### 3. Preparation of 1-(4'-fluorophenyl)-5-methoxy-1,4-dihydro-cinnoline-4-oxo-3-carboxylic acid

To 1.0 g (3.0 mmole) of the above methyl ester (from step 2) in 40 ml of THF there are added 2.0 g (10 eq. 85%) of KOH in 20 ml of MeOH and catalytic amount of 18-crown-6 at room temperature. The mixture is refluxed overnight. 0.9 g (92%) of crude product is prepared after acidifying with 1 N HCl.

IV(b)-IV(f) Following the general procedure of Example IV(a), Examples IV(b)-IV(f) were performed, using different solvents, various proportions of potassium hydroxide and either the methyl ester, as in Example IV(a), or the free acid as the starting material, all as set forth in Table 4, along with the purity and yields of the product where available.

**TABLE 4**

| **Summary Of Reaction Conditions And Results For Making 1-(4'-Fluorophenyl)-5-methoxy-1,4-dihydro-4-oxo-cinnoline-3-carboxylic Acid** | | | | | |
|---|---|---|---|---|---|
| RXN | Reactant | Eq. of KOH | Solvent | (%) Purity | (%) Yield |
| IV(b) | Ester | 15.0 | MeOH | 58 | - |
| IV(c) | Acid | 13.5 | p-dioxane | 50 | - |
| IV(d) | Ester | 15.0 | p-dioxane | - | - |
| IV(e) | Acid | 5.0 | THF (18-crown-6) | 86 | 98 |
| IV(f) | Acid | 8.0 | THF (18-crown-6) | 97 | 96 |

### Example V

### Preparation of 1-(4'-chlorophenyl)-5-methoxyethoxy-1,4-dihydro-4-oxo-cinnoline-3-carboxylic acid

### 1. Preparation of methyl 2,6-dichlorobenzoylacetoacetate

A slurry of anhydrous magnesium chloride (571 g, 6.0 moles) and methyl acetoacetate (697 g, 6.0 moles) in 3 l of acetonitrile was stirred in an ice-salt bath as 972 ml of pyridine (12 moles) was added at such a rate that the temperature did not exceed 5°C (addition time 2 hrs.). The cooling bath was removed and toluene (500 ml) was added at once followed by the addition of a solution of 1255 g of 2,6-dichlorobenzoyl chloride (6.0 moles) in 1.5 l of toluene over one hour (final temperature 25°C). The reaction was heated at reflux for 5 hours, and then allowed to cool over 14 hours to room temperature. The reaction was cooled in an ice bath to 0°C, and 400 ml of concentrated sulfuric acid (7.3 moles) was then added to the reaction mixture at a rate to maintain a temperature below 15°C, followed by 1.5 l of water. The mixture was stirred for 30 min. The organic layer was isolated and washed with 3 l of water. The acidic aqueous washes were back-extracted with 1 l of toluene. The product was extracted from the combined organic layers with an aqueous potassium carbonate solution (3.6 moles in 3 l water) and with a second potassiam carbonate solution (0.4 moles in 1 l water). The combined basic aqueous layers were washed with toluene (1 l) and acidified with concentrated hydrochloric acid. The resulting solid was isolated by filtration, washed with water (2 x 2 l), and dried under vacuum at 50°C to give 1008 g (58%) of a granular orange solid.

### 2. Preparation of methyl 3-(2',6'-dichlorophenyl)-2,3-dioxopropionate-2-(4''-chlorophenyl) hydrazone

A solution of 428 g of 4-chloroaniline (3.36 moles) in 2 l of ethanol (denatured, anhydrous) was stirred in an ice-salt bath as 586 ml of concentrated hydrochloric acid (7.04 mole) was added at such a rate that the temperature did not rise above 5°C. The solution was stirred as a solution of 238 g of sodium nitrite (3.56 moles) in 300 ml of water was added at such a rate that the temperature did not rise above 5°C. The resulting diazonium salt solution was stirred for 10 min. The stirring was stopped and the precipitate was allowed to settle. The diazonium salt was kept cold (5°C) until use.

A solution of 924 g of methyl 2,6-dichlorobenzoylacetoacetate (3.2 moles) from step 1 and potassium acetate (3 kg, 30.6 moles) in 13 l of 95% ethanol (denatured) was stirred in an ice-salt bath (temperature 5°C) as the above diazonium salt solution was added at once through a coarse filter. The reaction was stirred for 4 hrs. with cooling and allowed to warm to room temperature over 16 hrs. The precipitated product was diluted with 2 l of water and isolated by filtration. The solid was washed with water (2 x 2 l) and dried under reduced pressure at 50°C to give 1042 g (84%) of a yellow solid.

### 3. Preparation of methyl 5-chloro-1-(4'-chlorophenyl)-1,4-dihydro-4-oxo-cinnoline-3-carboxylate

A slurry of 376 g of potassium carbonate (2.7 moles) and 2 g of 18-crown-6 in 3.0 l of dimethylformamide was stirred and heated at 130°C as 1.0 kg of solid hydrazone (2.6 moles) from step 2 was added over 30 min. The reaction was heated at 135°C for 1 hr. The reaction was allowed to cool to 60°C over 3 hrs. and then cooled to 25°C in an ice bath. The thick mixture was poured into 6 l of cold water. The resulting slurry was stirred for 30 min. before the solid was isolated by filtration, washed with water (2 x 2 l), and dried under reduced pressure at 45°C to give 905 g (99%) of an off-white solid.

### 4. Preparation of 1-(4'-chlorophenyl)-1,4-dihydro-5-methoxyethoxy-4-oxo-cinnoline-3-carboxylic acid

A solution of 255 g of potassiam hydroxide (3.9 moles, pellets, 85+%) in 3.2 l of methoxyethanol was stirred at 45°C as 450 g of solid methyl phenylcinnoline-carboxylate(1.29 moles) from step 3 was added in portions. The reaction vessel was fitted with a distillation head and placed under reduced pressure (approx. 40 mmHg). The reaction was heated at 50°C for 1 hr., then at 65°C for 1 hr. to distill off methanol and some methoxyethanol (total volume removed 280 ml). The distillation head was replaced with a reflux condenser and the reaction was heated between 90°C and 110°C for 1 hr. The reaction was allowed to cool to 50°C before the addition of 1 liter of water and was cooled to 25°C before the resulting slurry was poured into 4 l of a cold 0.8 N hydrochloric acid solution (3.1 moles HCl). The resulting precipitate was isolated by filtration, washed with water (2 x 2 1), and dried under reduced pressure at 50°C to give 472 g (98%) of an off-white solid.

## Claims

1. Method for the preparation of a substituted cinnoline of the formula: 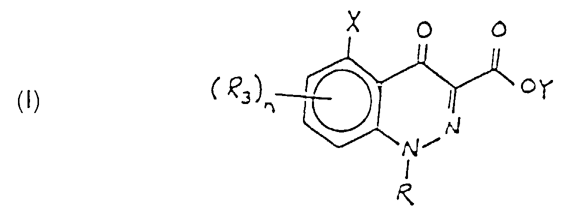 wherein
X represents a group of the formula OR₁ or NR₁R₂ wherein R₁ represents a C₁-C₄ alkyl group optionally substituted with a C₁-C₄ alkoxy group, 1 to 3 halogen atoms, or a carboxy or C₁-C₄ alkoxycarbonyl group; a C₂-C₄ alkenyl group optionally substituted with 1 to 3 halogen atoms; or a C₂-C₄ alkynyl group and R₂ represents H or an alkyl group;
Y is hydrogen, cation of a salt, C₁-C₂₂ linear alkyl or alkenyl containing up to four carbon-carbon double bonds, C₃-C₆ branched alkyl or alkenyl, C₁-C₄ alkoxyalkyl, cyclohexylmethyl, halogenated C₁-C₄ alkyl, phenyl, benzyl, -(CH₂CH₂O)ₘCH₂CH₃ in which m is an integer from 1 to 5, or -CH(CH₂OR₄)CH₂OR₅ or -CH₂CHOR₄CH₂OR₅ in which either R₄ or R₅ but not both represent a C₁-C₂₂ linear alkyl- or alkenylcarbonyl group containing up to four carbon-carbon double bonds and the other of R₄ or R₅ is H;
R represents C₁-C₄ alkyl, phenyl, naphtyl, or phenyl or naphtyl substituted with one to three substituents selected from the group consisting of halogen , CONH₂, C₁-C₄ alkyl or haloalkyl, C₁-C₄ alkoxy or haloalkoxy, and cyano; and
each R₃ is C₁-C₄ alkyl or haloalkyl, C₁-C₄ alkoxy or haloalkoxy, C₁-C₄ alkanoyl, cyano, nitro, hydroxy, or halo substituent which can be the same or different and n is an integer from 0 to 3 which comprises:
reacting at a temperature from about 60°C to about 90°C a compound of formula: 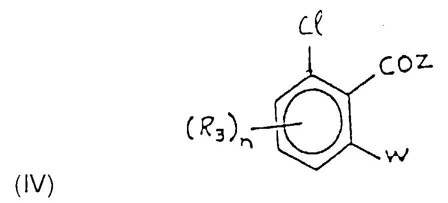 wherein
R₃ and n have the previously defined meanings;
W is either F or Cl; and
Z is H or Cl; with a compound of the formula 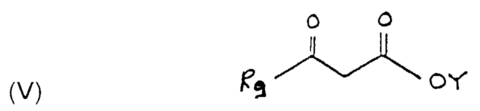
wherein R₉ is a hydrocarbon group, containing 10 or fewer carbons to form a trione of the formula: 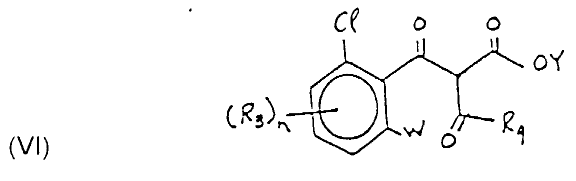
(1) converting said trione to a dione by heating at a temperature from about 50°C to about 100°C in an organic solvent, said dione compound having the formula: 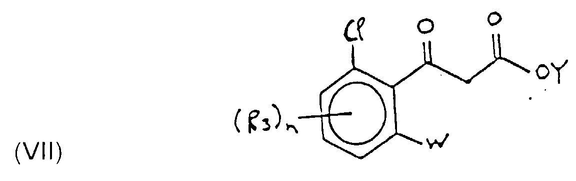 and reacting said dione with a diazonium salt of the formula:
RN⁺ ₂A⁻
wherein A⁻ is any counter anion, to form a hydrazone of the formula: 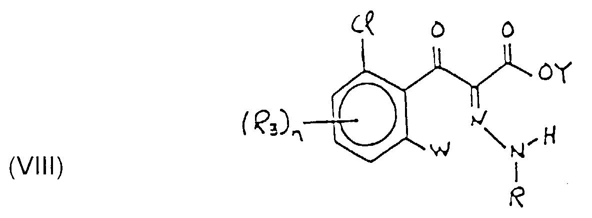 or
(2) converting said trione directly to the hydrazone of formula VIII by reacting said trione with said diazonium salt of the formula RN₂ ⁺A;⁻
causing said hydrazone to undergo a self-condensation reaction to produce a cinnoline precursor compound of the formula: 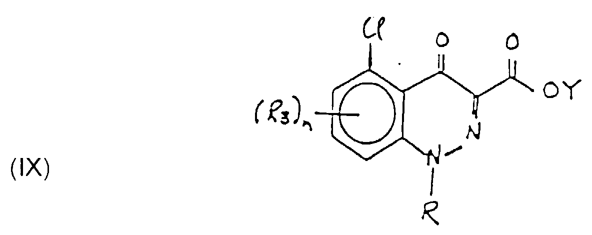 and reacting said cinnoline precursor compound with:
(1) a compound of the formula
R₁OH or R₁O⁻M⁺
wherein M⁺ is a metal cation, or
(2) a compound of the formula
NHR₁R₂
to form the desired compound of formula I.

2. Method according to claim 1, wherein R represents phenyl or phenyl substituted with one to three substituents consisting of halogen, trihalomethyl, C₁-C₄ alkoxy, C₁-C₄ alkyl, and cyano.

3. Method according to claim 1 or 2, wherein R is phenyl or phenyl substituted with a substituent consisting of 3', 4'-dichloro, 3',4'-difluoro, 4'-methyl, 4'-methoxy,4'-trifluoromethyl, 4'-iodio, 4'-chloro, 4'-fluoro, 4'-bromo, 3'-fluoro, 3'-chloro, 3'-trifluoromethyl, 2'-fluoro, 2'-chloro, 4'-trifluoromethoxy, and 4'-cyano.

4. Method according to claim 1 or 2, wherein R is phenyl or phenyl substituted with 4'-fluoro or 2'-fluoro, X is OCH₃, and Y is H or cation of a salt.

5. Method according to claim 1 or 2, wherein R is phenyl or phenyl substituted with 3';4'-difluoro, X is OCH₃, and Y is H or cation of a salt.

6. Method according to claim 1 or 2, wherein R is phenyl, X is OCH₂CH₂CH₃ or OCH(CH₃)₂ and Y is H or cation of a salt.

7. Method according to claim 1 or 2, wherein R is phenyl or phenyl substituted with 4'-chloro, X is OCH₂CH₂OCH₃, and Y is H or cation of a salt.

8. Method according to claim 1 or 2, wherein R is phenyl substituted with 4'-chloro, X is OCH(CH₃)CO₂H, and Y is H or cation of a salt.

9. Method according to claim 1 , wherein said compound of formula VI is a 2-(2',6'-dichlorobenzoyl)-3-keto-3-(C₁-C₄ alkyl) propanoic acid ester.

10. Compound of the formula 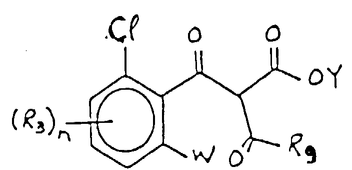 wherein:
W is either F or Cl
n is an integer from 0 to 3;
each R₃ independently represents a C₁-C₄ alkyl or haloalkyl, C₁-C₄ alkoxy or haloalkoxy, C₁-C₄ alkanoyl, cyano, nitro, hydroxy, or halo substituent,
R₉ is a hydrocarbon containing 10 or fewer carbons, and Y is H; an alkali metal ion; or a C₁-C₂₂ linear alkyl or alkenyl containing up to four carbon-carbon double bonds, C₃-C₆ branched alkyl or alkenyl, C₁-C₄ alkoxyalkyl, cyclohexylmethyl, halogenated C₁-C₄ alkyl, phenyl, benzyl,-(CH₂CH₂O)ₘCH₂CH₃ in which m is an integer from 1 to 5, or-CH(CH₂OR₄)CH₂OR₅ or -CH₂CHOR₄CH₂OR₅ in which either R₄ or R₅ but not both represent a C₁-C₂₂ linear alkyl- or alkenylcarbonyl group containing up to four carbon-carbon double bonds and the other of R₄ or R₅ is H, providing that such a compound cannot be:

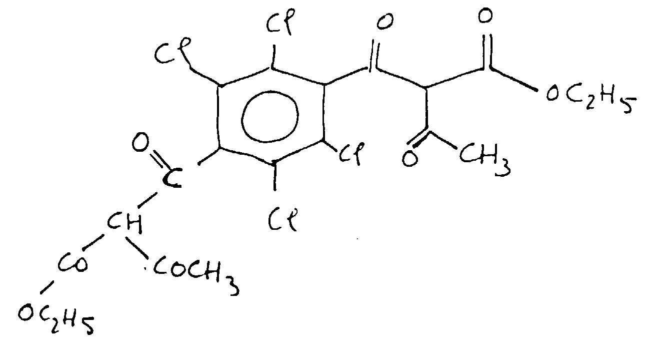

11. Compound according to claim 10, wherein R₉ is a C₁-C₄ hydrocarbon.

12. Compound according to claim 10, wherein said compound is a 2-(2',6'-dichlorobenzoyl)-3-keto-3-(C₁-C₄ alkyl )propanoic ester.

13. Compound according to claim 10, wherein said compound is 2-(2',6'-dichlorobenzoyl)-3-ketobutanoic acid ester.

## Patentansprüche

1. Verfahren zur Herstellung eines substituierten Cinnolins der Formel 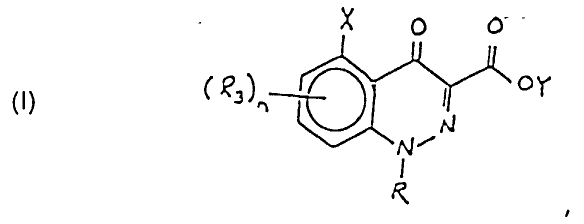 wobei
X eine Gruppe der Formel OR₁ oder NR₁R₂ darstellt, wobei R₁ eine C₁-C₄-Alkylgruppe, die gegebenenfalls mit einer C₁-C₄-Alkoxygruppe, 1 bis 3 Halogenatomen oder einer Carboxy- oder C₁-C₄-Alkoxycarbonylgruppe substituiert ist, eine C₂-C₄-Alkenylgruppe, die gegebenenfalls mit 1 bis 3 Halogenatomen substituiert ist, oder eine C₂-C₄-Alkinylgruppe darstellt und R₂ H oder eine Alkylgruppe darstellt,
Y Wasserstoff, das Kation eines Salzes, ein lineares C₁-C₂₂-Alkyl oder -Alkenyl mit bis zu vier Kohlenstoff-Kohlenstoff-Doppelbindungen, verzweigtes C₃-C₆-Alkyl oder -Alkenyl, C₁-C₄-Alkoxyalkyl, Cyclohexylmethyl, halogeniertes C₁-C₄-Alkyl, Phenyl, Benzyl, -(CH₂CH₂O)ₘCH₂CH₃, wobei m eine ganze Zahl von 1 bis 5 ist, oder -CH(CH₂OR₄)CH₂OR₅ oder -CH₂CHOR₄CH₂OR₅, wobei entweder R₄ oder R₅, aber nicht beide, eine lineare C₁-C₂₂-Alkyl- oder -Alkenylcarbonylgruppe mit bis zu vier Kohlenstoff-Kohlenstoff-Doppelbindungen darstellt und das jeweils andere, R₄ oder R₅, H ist,
R C₁-C₄-Alkyl, Phenyl, Naphthyl oder mit einem bis drei Substituenten, die aus der Gruppe ausgewählt sind, die aus Halogen, CONH₂, C₁-C₄-Alkyl oder -Halogenalkyl, C₁-C₄-Alkoxy oder -Halogenalkoxy und Cyan besteht, substituiertes Phenyl oder Naphthyl darstellt, und
jedes R₃ ein C₁-C₄-Alkyl- oder -Halogenalkyl-, C₁-C₄-Alkoxy- oder -Halogenalkoxy-, C₁-C₄-Alkanoyl-, Cyan-, Nitro-, Hydroxy- oder Halogensubstituent ist, die gleich oder verschieden sein können, und n eine ganze Zahl von 0 bis 3 ist,
umfassend:
Umsetzen einer Verbindung der Formel 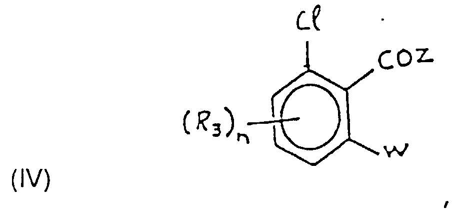 wobei R₃ und n dasselbe wie oben bedeuten, W entweder F oder Cl ist und Z H oder Cl ist, bei einer Temperatur von etwa 60°C bis etwa 90°C mit einer Verbindung der Formel 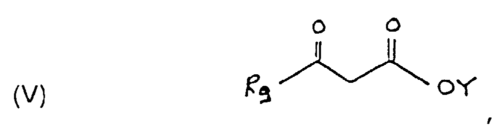 wobei R₉ eine Kohlenwasserstoffgruppe mit 10 oder weniger Kohlenstoffatomen ist, unter Bildung eines Trions der Formel 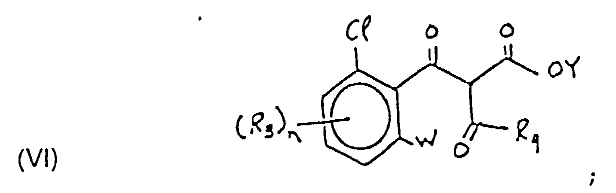
(1) Umsetzen des Trions zu einem Dion durch Erhitzen auf eine Temperatur von etwa 50°C bis etwa 100°C in einem organischen Lösungsmittel, wobei die Dionverbindung die Formel 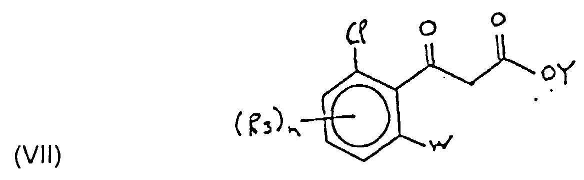 hat, und Umsetzen des Dions mit einem Diazoniumsalz der Formel
RN₂ ⁺A⁻,
wobei A⁻ irgendein Gegenanion ist, unter Bildung eines Hydrazons der Formel 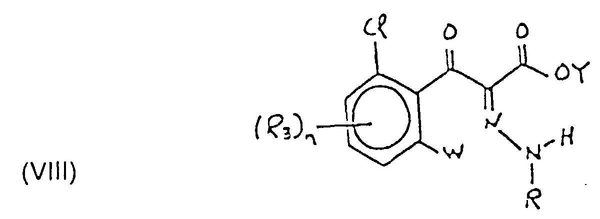 oder
(2) Umwandeln des Trions direkt in das Hydrazon der Formel VIII durch Umsetzen des Trions mit dem Diazoniumsalz der Formel RN₂ ⁺A⁻;
Durchführen einer Selbstkondensationsreaktion mit dem Hydrazon unter Bildung einer Cinnolinvorstufenverbindung der Formel 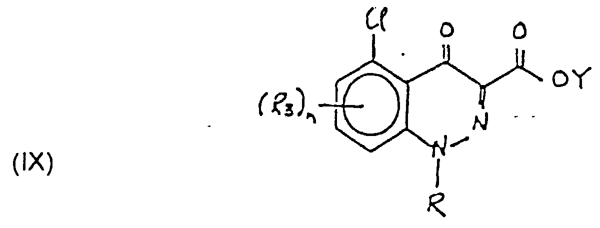 und Umsetzen der Cinnolinvorstufenverbindung mit
(1) einer Verbindung der Formel
R₁OH oder R₁O⁻M⁺,
wobei M⁺ ein Metallkation ist, oder
(2) einer Verbindung der Formel
NHR₁R₂
unter Bildung der gewünschten Verbindung der Formel I.

2. Verfahren gemäß Anspruch 1, wobei R Phenyl oder mit einem bis drei Substituenten, die aus Halogen, Trihalogenmethyl, C₁-C₄-Alkoxy, C₁-C₄-Alkyl und Cyan ausgewählt sind, substituiertes Phenyl darstellt.

3. Verfahren gemäß Anspruch 1 oder 2, wobei R Phenyl oder mit einem Substituenten, der aus 3',4'-Dichlor, 3',4'-Difluor, 4'-Methyl, 4'-Methoxy, 4'-Trifluormethyl, 4'-Iod, 4'-Chlor, 4'-Fluor, 4'-Brom, 3'-Fluor, 3'-Chlor, 3'-Trifluormethyl, 2'-Fluor, 2'-Chlor, 4'-Trifluormethoxy und 4'-Cyan ausgewählt ist, substituiertes Phenyl ist.

4. Verfahren gemäß Anspruch 1 oder 2, wobei R Phenyl oder mit 4'-Fluor oder 2'-Fluor substituiertes Phenyl ist, X OCH₃ ist und Y H oder das Kation eines Salzes ist.

5. Verfahren gemäß Anspruch 1 oder 2, wobei R Phenyl oder mit 3',4'-Difluor substituiertes Phenyl ist, X OCH₃ ist und Y H oder das Kation eines Salzes ist.

6. Verfahren gemäß Anspruch 1 oder 2, wobei R Phenyl ist, X OCH₂CH₂CH₃ oder OCH(CH₃)₂ ist und Y H oder das Kation eines Salzes ist.

7. Verfahren gemäß Anspruch 1 oder 2, wobei R Phenyl oder mit 4'-Chlor substituiertes Phenyl ist, X OCH₂CH₂OCH₃ ist und Y H oder das Kation eines Salzes ist.

8. Verfahren gemäß Anspruch 1 oder 2, wobei R mit 4'-Chlor substituiertes Phenyl ist, X OCH(CH₃)CO₂H ist und Y H oder das Kation eines Salzes ist.

9. Verfahren gemäß Anspruch 1, wobei die Verbindung der Formel VI ein 2-(2',6'-Dichlorbenzoyl)-3-keto-3-(C₁-C₄-alkyl)propansäureester ist.

10. Verbindung der Formel 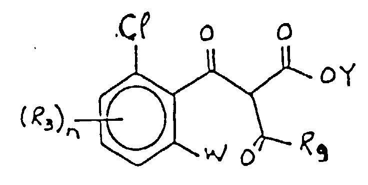 wobei
W entweder F oder Cl ist,
n eine ganze Zahl von 0 bis 3 ist,
jedes R₃ unabhängig einen C₁-C₄-Alkyl- oder -Halogenalkyl-, C₁-C₄-Alkoxy- oder -Halogenalkoxy-, C₁-C₄-Alkanoyl-, Cyan-, Nitro-, Hydroxy- oder Halogensubstituenten darstellt,
R₉ eine Kohlenwasserstoffgruppe mit 10 oder weniger Kohlenstoffatomen ist und
Y H, ein Alkalimetallion oder ein lineares C₁-C₂₂-Alkyl oder -Alkenyl mit bis zu vier Kohlenstoff-Kohlenstoff-Doppelbindungen, verzweigtes C₃-C₆-Alkyl oder -Alkenyl, C₁-C₄-Alkoxyalkyl, Cyclohexylmethyl, halogeniertes C₁-C₄-Alkyl, Phenyl, Benzyl, -(CH₂CH₂O)ₘCH₂CH₃, wobei m eine ganze Zahl von 1 bis 5 ist, oder -CH(CH₂OR₄)CH₂OR₅ oder -CH₂CHOR₄CH₂OR₅, wobei entweder R₄ oder R₅, aber nicht beide, eine lineare C₁-C₂₂-Alkyl- oder -Alkenylcarbonylgruppe mit bis zu vier Kohlenstoff-Kohlenstoff-Doppelbindungen darstellt und das jeweils andere, R₄ oder R₅, H ist,
mit der Maßgabe, daß es sich bei der Verbindung nicht um 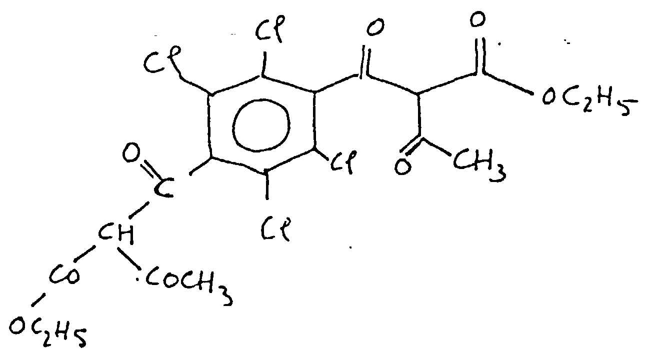 handelt.

11. Verbindung gemäß Anspruch 10, wobei R₉ eine C₁-C₄-Kohlenwasserstoffgruppe ist.

12. Verbindung gemäß Anspruch 10, wobei die Verbindung ein 2-(2',6'-Dichlorbenzoyl) -3-keto-3-(C₁-C₄-alkyl)propansäureester ist.

13. Verbindung gemäß Anspruch 10, wobei die Verbindung ein 2-(2',6'-Dichlorbenzoyl) -3-ketobutansäureester ist.

## Revendications

1. Un procédé pour la préparation d'une cinnoline substituée de formule : 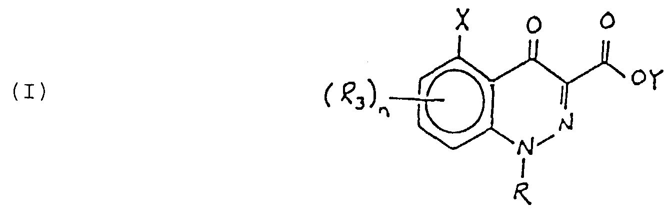 dans laquelle :
X représente un groupe de formule OR₁, ou NR₁R₂, où R₁ représente un groupe alkyle en C₁-C₄ éventuellement substitué avec un groupe alcoxy en C₁-C₄, 1 à 3 atomes d'halogène ou un groupe carboxy ou un groupe alcoxy carbonyle en C₁-C₄, un groupe alcényle en C₂-C₄, éventuellement substitué avec 1 à 3 atomes d'halogène ou un groupe alcynyle en C₂-C₄, et R₂ représente H ou un groupe alkyle ;
Y est l'hydrogène, un cation d'un sel, un groupe alkyle ou alcényle linéaire en C₁-C₂₂ renfermant jusqu'à quatre double liaisons carbone-carbone, un radical alkyle ou alcényle ramifié en C₃-C₆, un groupe alcoxy-alkyle en C₁-C₄, un groupe cyclohexylméthyle, un groupe alkyle en C₁-C₄ halogéné, un groupe phényle, un groupe benzyle, un groupe -(CH₂CH₂O)ₘCH₂CH₃ où m est un nombre entier de 1 à 5, ou -CH(CH₂OR₄)CH₂OR₅ ou -CH₂CHOR₄CH₂OR₅ où soit R₄ soit R₅ mais non les deux représentent un groupe alkyl- ou alcénylcarbonyle linéaire en C₁-C₂₂ renfermant jusqu'à quatre double liaisons carbone-carbone et l'autre radical R₄ ou R₅ est H;
R représente un groupe alkyle en C₁-C₄, phényle, naphtyle ou phényle ou naphtyle substitué avec 1 à 3 substituants choisis parmi le groupe comprenant un halogène, CONH₂, un radical haloalkyle ou alkyle en C₁-C₄, un radical haloalcoxy ou alcoxy en C₁-C₄ et cyano; et
chaque radical R₃ est un radical haloalkyle ou alkyle en C₁-C₄, un radical haloalcoxy ou alcoxy en C₁-C₄, un radical alcanoyle en C₁-C₄, cyano, nitro, hydroxy ou un substituant halo pouvant être identique ou différent et n est un nombre entier de 0 à 3,
qui consiste :
à faire réagir à une température d'environ 60°C à environ 90°C, un composé de formule : 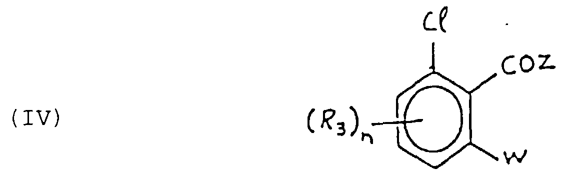 dans laquelle R₃ et n ont les significations définies ci-dessus ;
W est soit F soit Cl ; et
Z est H ou Cl ; avec un composé de formule : 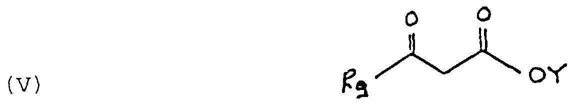
dans laquelle R₉ est un groupe hydrocarboné renfermant 10 atomes de carbone ou moins pour former une trione de formule : 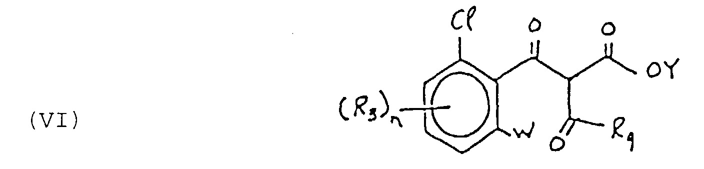
(1) à transformer ladite trione en une dione par chauffage à une température d'environ 50°C à environ 100°C dans un solvant organique, ledit composé de dione répondant à la formule : 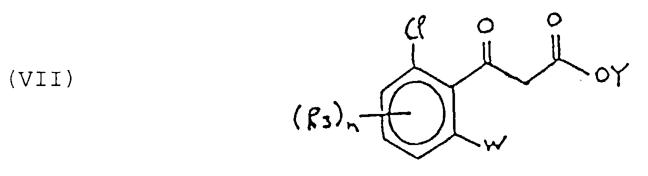 et à faire réagir ladite dione avec un sel de diazonium de formule :
RN₂ ⁺A⁻
dans laquelle A⁻ est n'importe quel contre-anion pour former une hydrazone de formule : 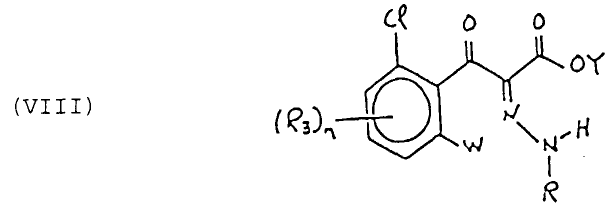
ou (2) à transformer ladite trione directement en l'hydrazone de formule VIII par réaction de ladite trione avec ledit sel de diazonium de formule RN₂ ⁺A⁻,
à provoquer que ladite hydrazone subisse une réaction d'auto-condensation pour produire un composé précurseur de cinnoline de formule : 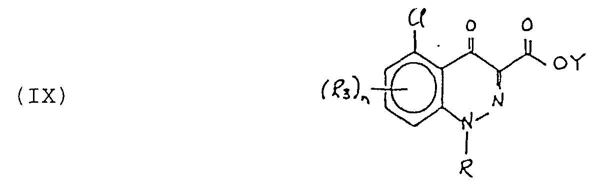 et à faire réagir ledit composé précurseur de cinnoline avec :
(1) un composé de formule
R₁OH ou R₁O⁻M⁺
dans laquelle M⁺ est un cation métallique, ou
(2) un composé de formule
NHR₁R₂
pour former le composé désiré de formule I.

2. Procédé selon la revendication 1, dans lequel R représente un radical phényle ou phényle substitué avec un à trois substituants se composant d'un halogène, d'un radical trihalométhyle, d'un radical alcoxy en C₁-C₄, d'un radical alkyle en C₁-C₄ et d'un radical cyano.

3. Procédé selon la revendication 1 ou 2, dans lequel R est un radical phényle ou phényle substitué avec un substituant se composant d'un radical 3', 4'-dichloro, 3',4'-difluoro, 4'-méthyle, 4'-méthoxy, 4'-trifluorométhyle, 4'-iodo, 4'-chloro, 4'-fluoro, 4'-bromo, 3'-fluoro, 3'-chloro, 3'-trifluorométhyle, 2'-fluoro, 2'-chloro, 4'-trifluorométhoxy et 4'-cyano.

4. Procédé selon la revendication 1 ou 2, dans lequel R est un radical phényle ou phényle substitué avec un radical 4'-fluoro ou 2'-fluoro, X est OCH₃ et Y est H ou un cation d'un sel.

5. Procédé selon la revendication 1 ou 2, dans lequel R est un radical phényle ou phényle substitué avec un radical 3', 4'-difluoro, X est OCH₃ et Y est H ou un cation d'un sel.

6. Procédé selon la revendication 1 ou 2, dans lequel R est un radical phényle, X est OCH₂CH₂CH₃ ou OCH(CH₃)₂ et Y est H ou un cation d'un sel.

7. Procédé selon la revendication 1 ou 2, dans lequel R est un radical phényle ou phényle substitué avec un radical 4'-chloro, X est OCH₂CH₂OCH₃ et Y est H ou un cation d'un sel.

8. Procédé selon la revendication 1 ou 2, dans lequel R est un radical phényle substitué avec un radical 4'-chloro, X est OCH(CH₃)CO₂H et Y est H ou un cation d'un sel.

9. Procédé selon la revendication 1, dans lequel ledit composé de formule VI est un ester d'acide 2-(2',6'-dichlorobenzoyl)-3-céto-3-(alkyle en C₁-C₄) propanoïque.

10. Composé de formule : 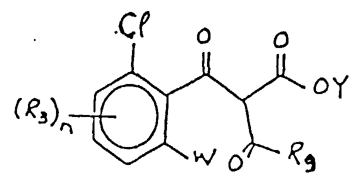 dans laquelle :
W est soit F soit Cl,
n est un nombre entier de 0 à 3,
chaque R₃ représente indépendamment un radical haloalkyle ou alkyle en C₁-C₄, un radical haloalcoxy ou alcoxy en C₁-C₄, un radical alcanoyle en C₁-C₄, cyano, nitro, hydroxy ou un substituant halo,
R₉ est un composé hydrocarboné renfermant 10 atomes de carbone ou moins, et Y est H, un ion de métal alcalin ou un radical alcényle ou alkyle linéaire en C₁-C₂₂ renfermant jusqu'à quatre doubles liaisons carbone-carbone, un radical alcényle ou alkyle ramifié en C₃-C₆, un radical alcoxyalkyle en C₁-C₄, cyclohexylméthyle, alkyle en C₁-C₄ halogéné, phényle, benzyle, -(CH₂CH₂O)ₘ CH₂CH₃ où m est un nombre entier de 1 à 5, où-CH(CH₂OR₄)CH₂OR₅ ou -CH₂CHOR₄CH₂OR₅ où soit R₄ soit R₅ mais non les deux représentent un groupe alkyl- ou alcénylcarbonyle ou linéaire en C₁-C₂₂ renfermant jusqu'à quatre doubles liaisons carbone-carbone et l'autre radical R₄ ou R₅ est H, à condition qu'un tel composé ne puisse pas être :

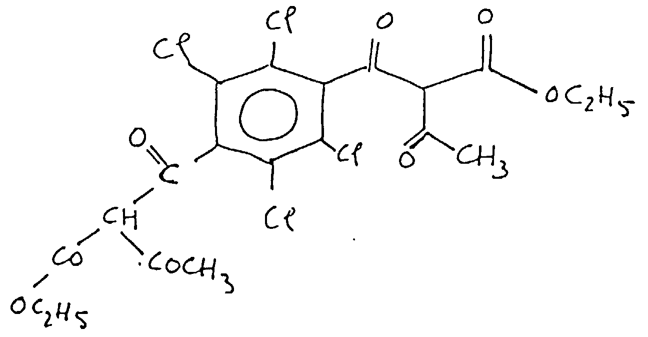

11. Composé selon la revendication 10, dans lequel R₉ est un hydrocarbure en C₁-C₄.

12. Composé selon la revendication 10, dans lequel ledit composé est un ester d'acide 2-(2',6'-dichlorobenzoyl)-3-céto-3-(alkyle en C₁-C₄) propanoïque.

13. Composé selon la revendication 10, dans lequel ledit composé est l'ester d'acide 2-(2',6'-dichlorobenzoyl)-3-cétobutanoïque.
